# EUROPEAN PATENT APPLICATION

(11) **EP 4 516 299 A1**
(43) Date of publication of application: **05.03.2025**
(21) Application number: 23796374.9
(22) Date of filing: 25.04.2023
(51) Int. Cl.: A61K 31/192, A61K 31/202, A61P 9/04

(54) **PHARMACEUTICAL COMPOSITION FOR TREATMENT OF HEART FAILURE WITH DIASTOLIC DYSFUNCTION**

(30) Priority: 28.04.2022 JP 2022073966
(71) Applicant: NATIONAL UNIVERSITY CORPORATION TOKAI NATIONAL HIGHER EDUCATION AND RESEARCH SYSTEM, Nagoya-shi, Aichi 464-8601 (JP); Toko Pharmaceutical Industries Co., Ltd., Tokyo 123-0865 (JP)
(72) Inventor: KATO Katsuhiro, Nagoya-shi, Aichi 464-8601 (JP); ENOMOTO Atsushi, Nagoya-shi, Aichi 464-8601 (JP); ISHIHARA Toshikazu, Nagoya-shi, Aichi 464-8601 (JP); MATSUMOTO Kotaro, Nagoya-shi, Aichi 464-8601 (JP); MUROHARA Toyoaki, Nagoya-shi, Aichi 464-8601 (JP)
(74) Representative: Kraus & Lederer PartGmbB
(86) International application number: PCT/JP2023/016268
(87) International publication number: WO 2023/210634

(57) **Abstract**

The present invention provides a pharmaceutical composition for treating heart failure with diastolic dysfunction, comprising a retinoid as an active ingredient. The heart failure with diastolic dysfunction may be heart failure with preserved left ventricular ejection fraction, heart failure with mid-range left ventricular ejection fraction, or heart failure with reduced left ventricular ejection fraction.

## Description

### TECHNICAL FIELD

The present invention relates to a pharmaceutical composition for treating heart failure with diastolic dysfunction.

### BACKGROUND ART

Heart failure with preserved ejection fraction (HFpEF) is heart failure primarily caused by left ventricular diastolic dysfunction, and accounts for about half of heart failure patients. Aging, obesity, diabetes mellitus, hypertension, and others have been reported to be risk factors for HFpEF. As super-aged society is approaching and the number of patients with lifestyle-related diseases is increasing, HFpEF is becoming an important public health issue, which is increasingly serious. HFpEF is now recognized as a systemic multiple organ syndrome including left ventricular diastolic dysfunction as well as various pathophysiological abnormalities. HFpEF is accompanied by high mortality. Five-year survival rate of patients hospitalized with HFpEF is about 35% on average, which is lower than that of many other cancers. QOL (Quality of life) of HFpEF patients is comparable to or lower than that of patients with heart failure with reduced ejection fraction (HFrEF).

For HFrEF or heart failure with mid-range ejection fraction (HFmrEF), the efficacy of four types of therapeutic drugs for heart failure (angiotensin receptor neprilysin inhibitors (ARNIs), β-blockers, mineralocorticoid receptor antagonists (MRAs), and sodium-dependent glucose co-transporter (SGLT2) inhibitors) has been reported, and administration of these drugs is recommended. Empagliflozin (SGLT2 inhibitor), which is clinically applied as a therapeutic drug for type 2 diabetes mellitus and chronic heart failure, is recently reported to be effective for HFpEF patients in clinical studies (Non patent literature 1). However, the efficacy of other therapeutic drugs for heart failure including ARNIs, β-blockers and MRAs for HFpEF has been reported not to be clear, and there is almost no effective therapy for HFpEF.

### CITATION LIST

### NON PATENT LITERATURE

Non patent literature 1: Anker S et al., N Engl J Med 2021; 385:1451-1461.

### SUMMARY OF INVENTION

### TECHNICAL PROBLEM

An object of the present invention is to provide a pharmaceutical composition useful for treatment of heart failure with diastolic dysfunction.

### SOLUTION TO PROBLEM

The present invention was made to solve the above problems and includes the following.
[1] A pharmaceutical composition for treating heart failure with diastolic dysfunction, comprising a retinoid as an active ingredient.
[2] The pharmaceutical composition according to the above [1], wherein the heart failure with diastolic dysfunction is any one selected from heart failure with preserved left ventricular ejection fraction, heart failure with mid-range left ventricular ejection fraction, and heart failure with reduced left ventricular ejection fraction.
[3] The pharmaceutical composition according to the above [1], wherein the heart failure with diastolic dysfunction is heart failure with preserved left ventricular ejection fraction.
[4] The pharmaceutical composition according to any one of the above [1] to [3], wherein the retinoid is tamibarotene or peretinoin.

### ADVANTAGEOUS EFFECTS OF INVENTION

The present invention provides a pharmaceutical composition useful for treatment of heart failure with diastolic dysfunction.

### BRIEF DESCRIPTION OF DRAWINGS

Fig. 1 shows the heart weight/tibia length ratio of each group after completion of drug administration in HFpEF model mice in Example 1.
Fig. 2 shows the left ventricular ejection fraction of each group before and after drug administration in Example 1. Panel (A) shows the results before start of drug administration, and panel (B) shows the results after completion of drug administration.
Fig. 3 shows the E/e' ratios of each group before and after drug administration in Example 1. Panel (A) shows the results of control group, panel (B) shows the results of tamibarotene administration group, and panel (C) shows the results of peretinoin administration group.
Fig. 4 shows the stiffness constant β of mice in each group after completion of drug administration in Example 1.
Fig. 5 shows the heart weight/tibia length ratio of each group after completion of drug administration in HFpEF model mice in Example 2.
Fig. 6 shows the left ventricular ejection fraction of each group before and after drug administration in Example 2. Panel (A) shows the results before start of drug administration, and panel (B) shows the results after completion of drug administration.
Fig. 7 shows the E/e' ratios of each group before and after drug administration in Example 2. Panel (A) shows the results of control group, panel (B) shows the results of tamibarotene administration group, and panel (C) shows the results of peretinoin administration group.
Fig. 8 shows the stiffness constant β of mice in each group after completion of drug administration in Example 2.
Fig. 9 shows Masson's trichrome staining of tissue sections prepared from the heart harvested after completion of drug administration in Example 2. Panel (A) shows a representative image observed under a microscope in control group. Panel (B) shows a representative image observed under a microscope in tamibarotene administration group. Panel (C) shows a representative image observed under a microscope in peretinoin administration group. Panel (D) shows comparison of fibrosis area among the groups in a high-power field.

### DESCRIPTION OF EMBODIMENTS

The present invention provides a pharmaceutical composition for treating heart failure with diastolic dysfunction, comprising a retinoid as an active ingredient. The subject for administration of the pharmaceutical composition of the invention is a patient who has developed heart failure with diastolic dysfunction. The heart failure with diastolic dysfunction may be any heart failure associated with diastolic dysfunction, and may include heart failure primarily caused by diastolic dysfunction, heart failure primarily caused by contractile dysfunction, and heart failure primarily caused by mild contractile dysfunction. The heart failure primarily caused by diastolic dysfunction may be heart failure with preserved left ventricular ejection fraction (HFpEF). The heart failure primarily caused by contractile dysfunction may be heart failure with reduced left ventricular ejection fraction (HFrEF). The heart failure primarily caused by mild contractile dysfunction may be heart failure with mid-range left ventricular ejection fraction (HFmrEF). Preferably, the patient is a patient with heart failure with preserved left ventricular ejection fraction (HFpEF).

Heart failure with diastolic dysfunction is induced by various factors that damage the main functions of the heart (epicardium and cardiac muscle, as well as endocardial diseases, valvular diseases, coronary artery diseases, aortal diseases, arrhythmia, etc.). Such factors include, but are not limited to, ischemic heart diseases (ischemic cardiomyopathy, stunning, hibernation, microcirculation damage), cardiomyopathy (hypertrophic cardiomyopathy, dilated cardiomyopathy, restrictive cardiomyopathy, arrhythmogenic right ventricular cardiomyopathy, noncompaction of ventricular myocardium, Takotsubo cardiomyopathy), myocarditis (viral myocarditis, bacterial myocarditis, rickettsial infection, Chagas' disease, etc.), immunological diseases (rheumatoid arthritis, systemic lupus erythematosus, polymyositis, mixed connective tissue disease, etc.), pregnancy (peripartum cardiomyopathy, postpartum cardiomyopathy, etc.), infiltrative diseases (sarcoidosis, amyloidosis, hemochromatosis, malignant tumor infiltration), endocrine and metabolic diseases (diabetes mellitus, thyroid diseases, Cushing's disease, pheochromocytoma, adrenal insufficiency, abnormal growth hormone secretion, etc.) congenital enzyme abnormalities (Fabry disease, Pompe disease, Hurler syndrome, Hunter syndrome, Gaucher disease), myopathy (myodystrophy, laminopathy), hypertension, valvular heart diseases and structural abnormalities of the heart (congenital valvular heart diseases, atrial septal defect, ventricular septal defect, other congenital diseases, aortic and mitral valve diseases, etc.), abnormalities of the epicardium such as constrictive pericarditis, abnormalities of the endocardium (eosinophilic endomyocardial diseases, endocardial fibroelastosis), high-output heart failure (anemia gravis, Paget's disease, arteriovenous shunt, pregnancy, beriberi heart, etc.), arrhythmia (atrial fibrillation, auricular tachycardia, ventricular tachycardia, sick sinus syndrome, atrioventricular block, etc.), etc. Heart failure with diastolic dysfunction is also induced by various other factors, including complex factors of unknown causes or idiopathic forms, such as cardiotoxic substances or other substances (habitual substances, such as alcohols, cocaine, amphetamine, or anabolic steroids; noble metals, such as copper, iron, lead, cobalt, or mercury; medicines, such as anticancer agents, immunosuppressants, antidepressants, antiarrhythmic drugs, non-steroidal anti-inflammatory drugs, or anesthetics; or radiation damage) or other diseases (chronic renal failure, sepsis, etc.).

Heart failure with diastolic dysfunction is determined by a physician based on echocardiography (cardiac ultrasound), cardiac catheterization, cardiac MRI examination, etc.

Echocardiography includes the following types of tests. Assessment of diastolic function by echocardiography is comprehensively performed by combining various types of markers as described below.

### (a) Left ventricular inflow velocity pattern (E/A) or transmitral flow velocity pattern (TMF)

When the dynamics of blood inflow from the left atrium to the left ventricle is recorded by pulsed Doppler echocardiography, early diastolic inflow velocity pattern (E-wave) and atrial systolic inflow velocity pattern (A-wave) are obtained. In the development of diastolic dysfunction, the ratio of the peak blood flow velocities (E/A) decreases, and the deceleration time (DT) of the E-wave is prolonged. When diastolic dysfunction is advanced and the left atrial pressure increases, the E-wave increases and the E/A ratio increases, and the velocity pattern changes to the "pseudo normal pattern," which appears to be similar to the normal pattern. When diastolic dysfunction is further advanced and the left atrial pressure further increases, the E/A ratio further increases and the velocity pattern changes to the "restrictive pattern." Decrease in the E/A ratio after Valsalva maneuver suggests diastolic dysfunction.

### (b) Early diastolic mitral annular velocity (e')

When the movement of the mitral valve annulus is recorded by tissue Doppler echocardiography, a systolic s' wave, an early diastolic e' wave, and an atrial systolic a' wave are obtained. The early diastolic e' wave correlates with the left ventricular relaxation time constant (Tau: τ), and decreases with impaired relaxation function.

### (c) E/e'

The ratio of the peak velocity of the left ventricular inflow velocity pattern (E-wave) and the peak velocity of the mitral annular velocity pattern (e' wave) (E/e') is not influenced by the left ventricular ejection fraction (LVEF) and positively correlates with the left atrial pressure (Ommen SR et al., Circulation 2000; 102: 1788-1794. PMID: 11023933).

### (d) Left atrial volume index (LAVI)

LAVI reflects chronic left atrial overload, and correlates with the degree of diastolic dysfunction (Takeda Y et al., J Card Fail 2009; 15: 68-77). The volume index is desirable, but the enlargement of the left atrial diameter also serves as a convenient marker for diastolic dysfunction in a clinical site.

### (e) Tricuspid regurgitation velocity (TRV)

### (f) Isovolumic relaxation time (IVRT)

IVRT is an interval between the closure of the aortic valve and the onset of inflow to the left ventricle.

### (g) Diastolic wall strain (DWS)

Reference: Pritchett AM et al., J Am Coll Cardiol 2005; 45: 87-92. PMID: 15629380.

### (h) Pulmonary venous flow velocity pattern (PVF)

PVF is composed of a systolic wave (PVS wave), a diastolic wave (PVD wave), and an atrial systolic wave (PVA wave). Abnormal left ventricular relaxation reduces the PVD wave. When the left ventricular end-diastolic pressure significantly increases, the PVA wave increases and its duration is prolonged. When the left ventricular systolic function decreases and the left ventricular end-diastolic pressure increases, the PVS wave decreases resulting in a PVS < PVD pattern.

### (i) Left ventricular flow propagation velocity (Vp)

Vp is measured by recording the blood flow from the mitral orifice to the center of the left ventricle using color M-mode echocardiography. Vp is reduced with decreases in the left ventricular relaxation and the left ventricular compliance.

### (j) Others

Left ventricular septal hypertrophy or posterior wall hypertrophy can also be used as markers for convenient morphological characterization in a clinical site.

Diastolic function can also be assessed by recording the left ventricular pressure by cardiac catheterization. Cardiac catheterization includes the following types of tests.
(a) Left ventricular relaxation time constant (Tau: τ)
   Prolonged LV relaxation time constant indicates delayed LV relaxation.
(b) Stiffness constant as a marker for left ventricular stiffness
   Chamber stiffness (K)
   Stiffness constant β
(c) End-diastolic pressure-volume relation (EDPVR)
(d) Peak negative dP/dt: the peak rate of LV pressure fall.
(e) dV/dP (change in pressure/volume): LV compliance.

Diastolic function can also be assessed by cardiac MRI examination. Cardiac MRI examination includes the following types of tests.
(a) Cardiac mass (Left Ventricular (LV) mass) and cardiac hypertrophy
(b) Left atrial volume and its functional assessment
(c) Peak filling rates and time to peak filling calculated from LV time-volume filling curves.
(d) E/A, E/e', and pulmonary venous flow velocity pattern
(e) Extra-cellular volume (ECV): assessment of fibrosis of cardiac tissue using T1 mapping.

The retinoid serving as an active ingredient of the pharmaceutical composition of the invention includes, for example, acitretin, adapalene, AGN194204 (also called IRX4204, NRX194204, and VTP 194204), AGN195183, alitretinoin (also called 9-cis-retinoic acid), amsilarotene, AM580, bexarotene, bisphenol A diglycidyl ether (also called BADGE), fenretinide, 4-hydroxyretinoic acid, isotretinoin (also called 13-cis-retinoic acid), LG268, LGD1550, peretinoin, retinyl acetate, tamibarotene (also called AM80), tazarotene, tretinoin (also called all-trans-retinoic acid (ATRA), TTNPB, etc. The retinoid is preferably tamibarotene or peretinoin. The term "retinoid" as used herein includes vitamin A derivatives, vitamin A analogs, retinoic acid receptor (RAR) agonists, and retinoid X receptor (RXR) agonists, but excludes retinoic acid receptor (RAR) antagonists or retinoid X receptor (RXR) antagonists.

The retinoid may be in the form of a salt, preferably in the form of a pharmaceutically acceptable salt. Examples of such a salt include a salt with an acid, such as hydrochloric acid, sulfuric acid, lactic acid, tartaric acid, maleic acid, fumaric acid, oxalic acid, malic acid, citric acid, oleic acid, palmitic acid, nitric acid, phosphoric acid, trifluoroacetic acid, methanesulfonic acid, benzenesulfonic acid, or p-toluenesulfonic acid; a salt of an alkali metal or an alkaline earth metal, such as sodium, potassium, or calcium, or a hydroxide of an aluminum, or a salt of a carbonate; or a salt of triethylamine, benzylamine, diethanolamine, t-butylamine, dicyclohexylamine, arginine, etc.

The retinoid used in the pharmaceutical composition of the invention can be formulated by appropriately combining the retinoid with a pharmaceutically acceptable carrier or additive in accordance with a known production method for pharmaceutical formulations (e.g., the methods described in the Japanese pharmacopoeia, etc.). Specific examples of such a pharmaceutical formulation include oral or parenteral formulations, such as tablets (including sugar-coated tablets, film-coated tablets, sublingual tablets, orally disintegrating tablets, buccal tablets, etc.), pills, powders, granules, capsules (including soft capsules and microcapsules), troches, syrups, solutions, emulsions, suspensions, controlled release formulations (e.g., fast release formulations, sustained release formulations, sustained release microcapsules, etc.), aerosols, films (e.g., orally disintegrating films, oral mucosal adhesive films, etc.), injections (e.g., subcutaneous injection, intravenous injection, intramuscular injection, intraperitoneal injection, etc.), intravenous infusions, transdermal formulations, ointments, lotions, patches, suppositories (e.g., rectal suppository, vaginal suppository, etc.), pellets, transnasal formulations, transpulmonary formulations (inhalants), eye drops, etc. The blending ratio of a carrier or additive may determined as appropriate based on the amount usually used in the pharmaceutical field. The carrier or additive that can be blended is not limited to a particular one, and examples thereof include various types of carriers, such as water, physiological saline, other aqueous solvents, or aqueous or oily bases; and various types of additives, such as excipients, binders, pH adjusting agents, disintegrants, absorption enhancers, lubricants, colorants, flavor improvers and fragrances.

Additives that can be blended into tablets, capsules, etc., may include, for example, binders such as gelatin, corn starch, tragacanth, or gum arabic; excipients such as crystalline cellulose; swelling agents such as corn starch, gelatin, or alginic acid; lubricants such as magnesium stearate; sweeteners such as sucrose, lactose, or saccharin; flavoring agents such as peppermint, wintergreen oil, or cherry; etc. When the formulation unit is a capsule, a liquid carrier such as fats and oils can be further blended into the formulation, in addition to the ingredients of the above types. A sterile composition for injection can be prepared according to conventional formulation procedures (for example, by dissolving or suspending the active ingredient in a solvent, such as water for injection or a natural vegetable oil). An aqueous liquid for injection may be, for example, physiological saline or an isotonic solution containing glucose or other auxiliary agents (e.g., D-sorbitol, D-mannitol, sodium chloride, etc.). The aqueous liquid for injection may further contain an appropriate solubilizing agent, including, for example, alcohols (ethanol, etc.), polyalcohols (propylene glycol, polyethylene glycol, etc.), and nonionic surfactants (polysorbate 80, HCO-50, etc.). An oily liquid for injection may be, for example, sesame oil, soybean oil, or the like, and may further contain a solubilizing agent such as benzyl benzoate and benzyl alcohol. Additionally, the liquid for injection may further contain a buffering agent (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc.

Retinoids have low toxicity to humans or other mammals (for example, rats, mice, rabbits, sheep, pigs, cows, cats, dogs, monkeys, etc.), and can be safely administered to these mammals. The amount of the retinoid contained in the formulation of the invention may vary depending on the dosage form, the mode of administration, the carrier to be used, etc., but may be typically 0.01 to 100% (w/w), preferably 0.1 to 95% (w/w), relative to the total amount of the formulation.

The dosage of the retinoid may vary depending on the subject of administration, the symptoms, the route of administration, etc. For oral administration, the dosage for a human with a body weight of about 60 kg is, for example, typically about 0.01 to 1000 mg per day, preferably about 0.1 to 500 mg per day, and more preferably about 0.5 to 100 mg per day. For parenteral administration, a single dose may vary depending on the conditions and symptoms of a patient, the mode of administration, etc. For example, for an injection, typically, the retinoid is intravenously administered at a single dose of about 0.01 to 1000 mg per kg body weight, preferably about 0.01 to 500 mg per kg body weight, and more preferably about 0.01 to 20 mg per kg body weight. The total daily dosage may be administered in a single dose or several divided doses.

The pharmaceutical composition of the invention may be used in combination with an additional therapeutic drug for heart failure. Examples of the additional therapeutic drug for heart failure include angiotensin converting enzyme (ACE) inhibitors, angiotensin II receptor antagonists (ARBs), aldosterone antagonists, β-blockers, mineralocorticoid receptor antagonists (MRAs), angiotensin receptor neprilysin inhibitors (ARNIs), sodium-dependent glucose co-transporter (SGLT2) inhibitors, I_{f} channel inhibitors (HCN channel blockers), soluble guanylate cyclase (sGC) stimulators, loop diuretics, thiazide diuretic drugs, vasopressin V2 receptor antagonists, digitalis, cardiotonic drugs, etc.

The pharmaceutical composition of the invention may be used in combination with therapy using a percutaneous cardiopulmonary support device (PCPS), an intra-aortic balloon pump (IABP), or a ventricular assist device (VAD), or may be used in combination with cardiac resynchronization therapy using a mechanical device, such as a biventricular pacemaker.

The term "used in combination with" as used herein means that the duration of application of the retinoid overlaps with the duration of application of an additional therapeutic drug or therapy for heart failure, and the retinoid is not necessarily required to be administered simultaneously with the additional therapeutic drug or therapy for heart failure. The term may mean that the pharmaceutical composition of the invention is administered to a patient who has already received an additional therapeutic drug for heart failure. The dosage of the additional therapeutic drug for heart failure may be determined according to the clinical dosage, and may be selected as appropriate for the subject for administration, the age and body weight of the subject for administration, the symptoms, the dosage form, the mode of administration, etc.

The present invention also includes the following.
(1) A method for treating heart failure with diastolic dysfunction, comprising administering a retinoid to a patient with heart failure with diastolic dysfunction.
(2) A retinoid for use in treatment of heart failure with diastolic dysfunction.
(3) Use of a retinoid for production of a pharmaceutical composition for treating heart failure with diastolic dysfunction.

### EXAMPLES

The present invention will be described in more detail below with reference to Examples, but the present invention is not limited thereto.

### Example 1: Effect of tamibarotene or peretinoin on HFpEF model mice

### (1) Experimental method

### (1-1) Production of HFpEF model mice

HFpEF model mice are generated by the method reported by Schiattarella et al. (Nature. 568(7752):351-356. 2019). Specifically, male mice at an age of 8 to 10 weeks are given High Fat Diet (Rodent Diet with 60% kcal% fat, D12492, Research Diets Inc.) and drinking water containing a dissolved nitric oxide synthase inhibitor (Nω-nitro-L-arginine methyl ester hydrochloride (L-NAME), N5751, Sigma) (at a concentration of 0.5 g/L), and kept for five weeks. Various measurements are performed to confirm the establishment of an HFpEF model.

### (1-2) Drug administration

The following drugs are administered to mouse groups while mice are continued to be given the high-fat diet and the drinking water containing the dissolved nitric oxide synthase inhibitor. AM80 (Cat. No. 3507, TOCRIS) is orally administered to tamibarotene administration group via oral gavage at a single dose of 3 mg/kg once a day. Peretinoin (Cat. No. HY-100008, MedChemExpress) is orally administered to peretinoin administration group via oral gavage at a single dose of 0.3 mg/body once a day. DMSO as a vehicle is orally administered to control group via oral gavage once a day. Administration is continued for five weeks, and various measurements are performed five weeks after the initial drug administration.

### (1-3) Echography

Transthoracic echocardiography is performed on mice under inhalational anesthesia with isoflurane. Transmitral flow velocity pattern (TMF) is measured by pulsed Doppler echocardiography using vevo 3100 (FUJIFILM VisualSonics) equipped with a 30 MHz probe for small animals (MX-400) to determine the early diastolic inflow velocity pattern (E-wave). Early diastolic mitral annular velocity (e') is measured by tissue Doppler echocardiography. The transmitral E velocity is divided by the e' velocity to calculate an E/e' ratio. LV end-systolic diameter and LV end-diastolic diameter are measured by M-mode echocardiography to calculated an LV ejection fraction (EF%).

### (1-4) Measurement of left ventricular function using conductance catheter

To investigate the left ventricular function and other functions of HFpEF model mice, pressure-volume (PV) loop analysis is performed on mice under inhalational anesthesia with isoflurane using a conductance catheter (SPR839; MPVS-UIltra Pressure Volume System; Millar Instruments). The measurement is performed by the methods reported by Pacher et al. (Nat Protoc. 3(9): 1422-1434, 2008) and Cingolani et al. (Am J Physiol Heart Circ Physiol 301: H2198-H2206, 2011). PV loops are measured under various loading conditions by transiently occluding the inferior vena cava, and the EDPVR (end-diastolic pressure-volume relationship) is calculated to determine the stiffness constant β.

### (1-5) Measurement of heart weight

Mice are euthanized after the catheterization, and the heart is harvested for weight measurement. The tibia is also harvested simultaneously for length measurement.

### (2) Results

### (2-1) Heart weight/tibia length ratios

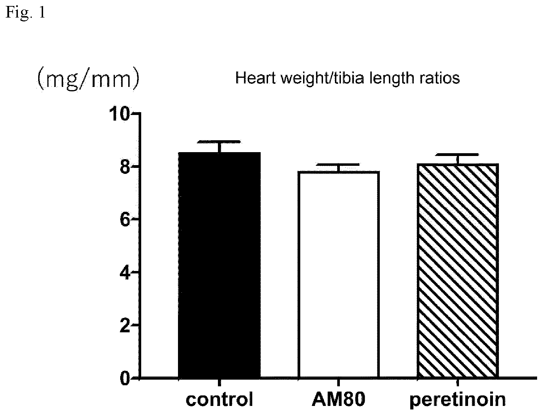

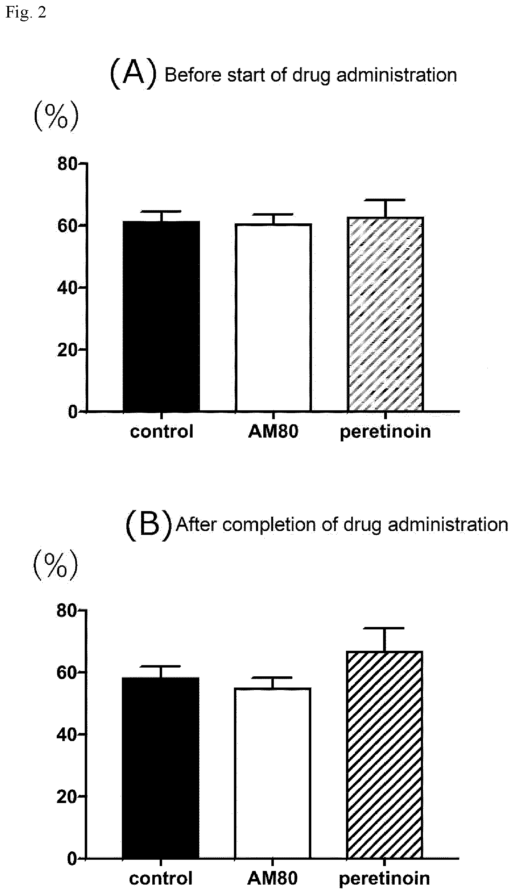

Fig. 1 shows the heart weight/tibia length ratios after completion of drug administration. The data represent the mean ± SEM (control group: n = 11, tamibarotene administration group: n = 9, peretinoin administration group: n = 7). In the figure, control indicates control group, AM80 indicates tamibarotene administration group, and peretinoin indicates peretinoin administration group (the same applies to Figs. 2 to 5). The results shows no significant difference among the groups.

### (2-2) Left ventricular ejection fractions

Fig. 2 shows the left ventricular ejection fractions before and after drug administration. Panel (A) shows the results before start of drug administration, and panel (B) shows the results after completion of drug administration. The data represent the mean ± SEM (control group: n = 11, tamibarotene administration group: n = 9, peretinoin administration group: n = 7). The results shows no difference in the left ventricular ejection fractions before and after drug administration.

### (2-3) E/e' ratios

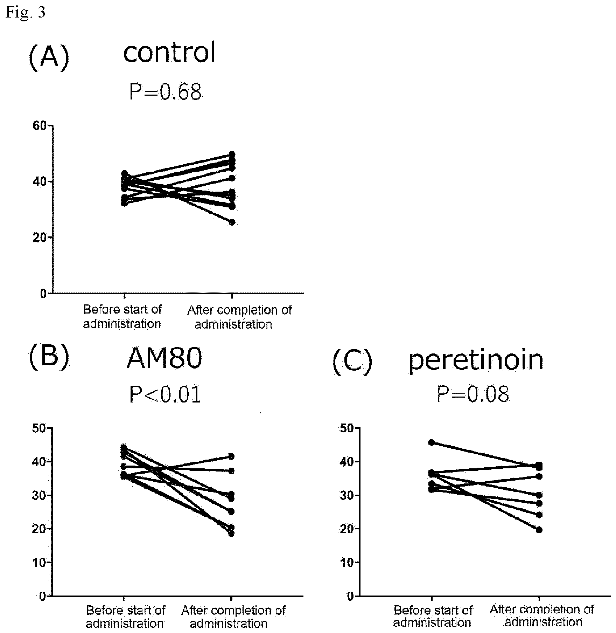

Fig. 3 shows the E/e' ratios before and after drug administration. Panel (A) shows the results of control group (n = 11), panel (B) shows the results of tamibarotene administration group (n = 9), and panel (C) shows the results of peretinoin administration group (n = 7). The results indicate that the E/e' ratios in tamibarotene and peretinoin administration groups after completion of drug administration are significantly reduced as compared with those before start of drug administration.

### (2-4) Stiffness constant β

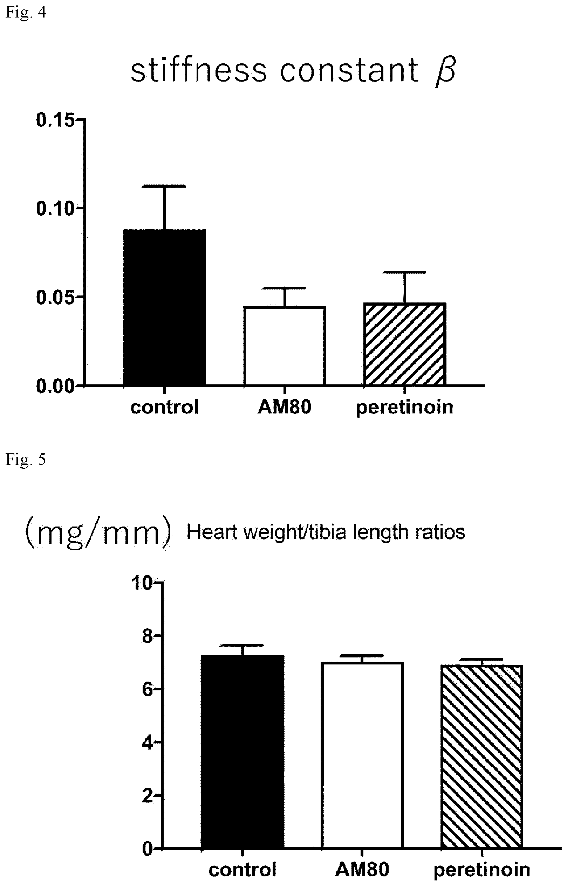

Fig. 4 shows the stiffness constant β. The data represent the mean ± SEM (control group: n = 6, tamibarotene administration group: n = 5, peretinoin administration group: n = 5). These results indicate that the stiffness constant β in tamibarotene and peretinoin administration groups is reduced as compared with that of control group.

### Example 2: Effect of tamibarotene or peretinoin on HFpEF model mice

### (1) Experimental method

### (1-1) Production of HFpEF model mice

HFpEF model mice are generated by the method reported by Schiattarella et al. (Nature. 568(7752):351-356. 2019). Specifically, male mice at an age of 8 to 10 weeks are given High Fat Diet (Rodent Diet with 60% kcal% fat, D12492, Research Diets Inc.) and drinking water containing a dissolved nitric oxide synthase inhibitor (Nω-nitro-L-arginine methyl ester hydrochloride (L-NAME), N5751, Sigma) (at a concentration of 0.5 g/L), and kept for five weeks. Various measurements are performed to confirm the establishment of an HFpEF model.

### (1-2) Drug administration

The following drugs are administered to mouse groups while mice are continued to be given the high-fat diet and the drinking water containing the dissolved nitric oxide synthase inhibitor. AM80 (Cat. No. 3507, TOCRIS) is orally administered to tamibarotene administration group via oral gavage at a single dose of 3 mg/kg once a day. Peretinoin (Cat. No. HY-100008, MedChemExpress) is orally administered to peretinoin administration group via oral gavage at a single dose of 10 mg/kg once a day. DMSO as a vehicle is orally administered to control group via oral gavage once a day. Administration is continued for five weeks, and various measurements are performed five weeks after the initial drug administration.

### (1-3) Echography

Transthoracic echocardiography is performed on mice under inhalational anesthesia with isoflurane. Transmitral flow velocity pattern (TMF) is measured by pulsed Doppler echocardiography using vevo 3100 (FUJIFILM VisualSonics) equipped with a 30 MHz probe for small animals (MX-400) to determine the early diastolic inflow velocity pattern (E-wave). Early diastolic mitral annular velocity (e') is measured by tissue Doppler echocardiography. The transmitral E velocity is divided by the e' velocity to calculate an E/e' ratio. LV end-systolic diameter and LV end-diastolic diameter are measured by M-mode echocardiography to calculated an LV ejection fraction (EF%).

### (1-4) Measurement of left ventricular function using conductance catheter

To investigate the left ventricular function and other functions of HFpEF model mice, pressure-volume (PV) loop analysis is performed on mice under inhalational anesthesia with isoflurane using a conductance catheter (PVR-1045; MPVS-UIltra Pressure Volume System; Millar Instruments). The measurement is performed by the methods reported by Pacher et al. (Nat Protoc. 3(9): 1422-1434, 2008) and Cingolani et al. (Am J Physiol Heart Circ Physiol 301: H2198-H2206, 2011). PV loops are measured under various loading conditions by transiently occluding the inferior vena cava, and the EDPVR is calculated to determine the stiffness constant β.

### (1-5) Measurement of heart weight

Mice are euthanized after the catheterization, and the heart is harvested for weight measurement. The tibia is also harvested simultaneously for length measurement.

### (1-6) Histological staining

Tissue specimens are prepared from the harvested heart, and stained with Masson's trichrome staining. The tissue fibrosis is photographed, and the images are analyzed using Image J for assessment.

### (2) Results

### (2-1) Heart weight/tibia length ratios

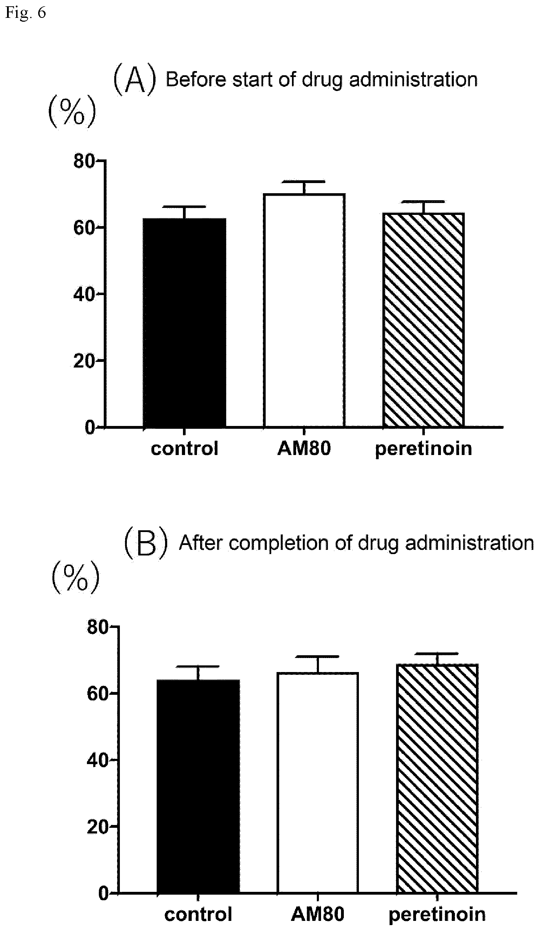

Fig. 5 shows the heart weight/tibia length ratios after completion of drug administration. The data represent the mean ± SEM (control group: n = 8, tamibarotene administration group: n = 8, peretinoin administration group: n = 8). In the figure, control indicates control group, AM80 indicates tamibarotene administration group, and peretinoin indicates peretinoin administration group (the same applies to Figs. 6 to 9). The results shows no significant difference among the groups.

### (2-2) Left ventricular ejection fractions

Fig. 6 shows the left ventricular ejection fractions before and after drug administration. Panel (A) shows the results before start of drug administration, and panel (B) shows the results after completion of drug administration. The data represent the mean ± SEM (control group: n = 9, tamibarotene administration group: n = 9, peretinoin administration group: n = 9). The results shows no difference in the left ventricular ejection fractions before and after drug administration.

### (2-3) E/e' ratios

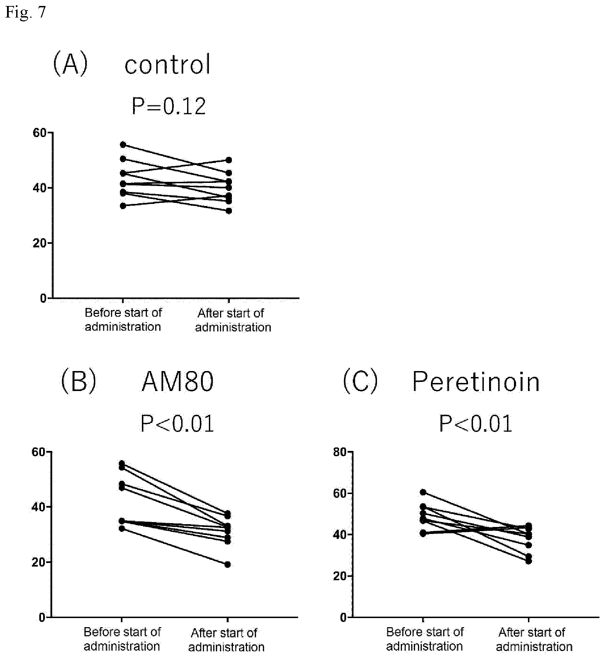

Fig. 7 shows the E/e' ratios before and after drug administration. Panel (A) shows the results of control group (n = 9), panel (B) shows the results of tamibarotene administration group (n = 9), and panel (C) shows the results of peretinoin administration group (n = 9). The results indicate that the E/e' ratios in tamibarotene and peretinoin administration groups after completion of drug administration are significantly reduced as compared with those before start of drug administration.

### (2-4) Stiffness constant β

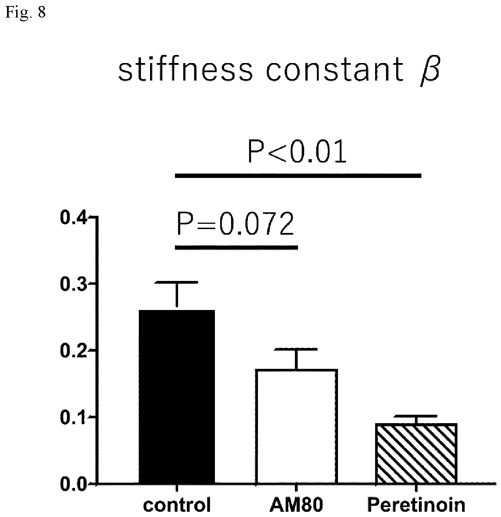

Fig. 8 shows the stiffness constant β. The data represent the mean ± SEM (control group: n = 8, tamibarotene administration group: n = 6, peretinoin administration group: n = 8). These results indicate that the stiffness constant β in tamibarotene and peretinoin administration groups is significantly reduced as compared with that of control group.

### (2-5) Masson's trichrome staining

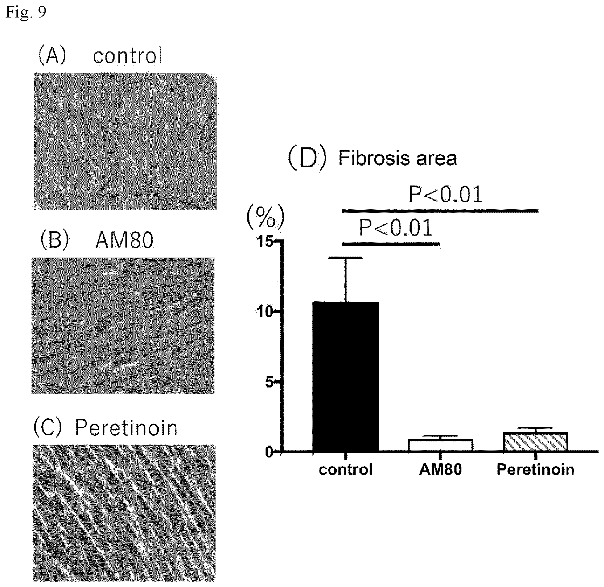

Masson's trichrome staining is shown in Fig. 9. Panel (A) shows a representative image observed under a microscope in control group. Panel (B) shows a representative image observed under a microscope in tamibarotene administration group. Panel (C) shows a representative image observed under a microscope in peretinoin administration group. Panel (D) shows comparison of fibrosis area among the groups in a high-power field (400-fold). The results are from control group (n = 4), tamibarotene administration group (n = 4), and peretinoin administration group (n = 4). The data represent the mean ± SEM. The results indicate that tissue fibrosis in tamibarotene and peretinoin administration groups after completion of drug administration is significantly reduced as compared with that of control group.

The results from Examples 1 and 2 as shown above indicate that both of tamibarotene and peretinoin exhibit improving effect on the diastolic function of the HFpEF model mice.

The present invention is not limited to each of the embodiments and Examples as described above, and various modifications are possible within the scope of the claims. Embodiments obtainable by appropriately combining the technical means disclosed in the different embodiments of the present invention are also included in the technical scope of the present invention. The scientific literature and the patent literature cited herein are hereby incorporated by reference in their entirety.

## Claims

1. A pharmaceutical composition for treating heart failure with diastolic dysfunction, comprising a retinoid as an active ingredient.

2. The pharmaceutical composition according to claim 1, wherein the heart failure with diastolic dysfunction is any one selected from heart failure with preserved left ventricular ejection fraction, heart failure with mid-range left ventricular ejection fraction, and heart failure with reduced left ventricular ejection fraction.

3. The pharmaceutical composition according to claim 1, wherein the heart failure with diastolic dysfunction is heart failure with preserved left ventricular ejection fraction.

4. The pharmaceutical composition according to any one of claims 1 to 3, wherein the retinoid is tamibarotene or peretinoin.
